# EUROPEAN PATENT SPECIFICATION

(11) **EP 2 069 792 B1**
(45) Date of publication and mention of the grant of the patent: **17.07.2013**
(21) Application number: 07804286.8
(22) Date of filing: 14.09.2007
(51) Int. Cl.: G01N 33/569, C07K 14/35

(54) **METHOD AND KIT FOR DETECTING IF AN INDIVIDUAL IS SUSCEPTIBLE TO PROGRESS TO AN ACTIVE MYCOBACTERIAL DISEASE**
VERFAHREN UND KIT ZUM NACHWEIS DER ANFÄLLIGKEIT EINES INDIVIDUUMS FÜR DAS FORTSCHREITEN EINER MYKOBAKTERIELLEN ERKRANKUNG ZU EINEM AKTIVEN ZUSTAND
METHODE ET KIT POUR DETECTER SI UN INDIVIDU EST SUSCEPTIBLE DE PROGRESSER VERS UNE FORME ACTIVE D'UNE MALADIE MYCOBACTERIENNE

(30) Priority: 14.09.2006 GB 0618127
(43) Date of publication of application: 17.06.2009
(73) Proprietor: Lalvani, Ajit, Oxford, OX2 7ES (GB)
(72) Inventor: Lalvani, Ajit, Oxford, OX2 7ES (GB); Millington, Kerry, London, N8 9RU (GB)
(74) Representative: Muir, Benjamin M. J.
(86) International application number: PCT/GB2007/003498
(87) International publication number: WO 2008/032092

(56) References cited:
- WO-A-2004/005925
- WO-A-2005/080990
- WO-A-2005/090988
- SHAMS HOMAYOUN ET AL: "Enzyme-linked immunospot and tuberculin skin testing to detect latent tuberculosis infection" AMERICAN JOURNAL OF RESPIRATORY AND CRITICAL CARE MEDICINE, vol. 172, no. 9, November 2005 (2005-11), pages 1161-1168, XP002466743 ISSN: 1073-449X
- MUSTAFA ET AL: "Recombinant and synthetic peptides to identify Mycobacterium tuberculosis antigens and epitopes of diagnostic and vaccine relevance" TUBERCULOSIS, ELSEVIER, GB, vol. 85, no. 5-6, September 2005 (2005-09), pages 367-376, XP005156237 ISSN: 1472-9792
- COCKLE P J ET AL: "Identification of novel Mycobacterium tuberculosis antigens with potential as diagnostic reagents or subunit vaccine candidates by comparative genomics" INFECTION AND IMMUNITY, AMERICAN SOCIETY FOR MICROBIOLOGY. WASHINGTON, US, vol. 70, no. 12, December 2002 (2002-12), pages 6996-7003, XP002252152 ISSN: 0019-9567

## Description

### Field of the Invention

The invention relates to a prognostic method.

### Background to the Invention

The tuberculin skin test (TST) has hitherto been the only prognostic marker for active tuberculosis (TB) in people with recent TB exposure. Larger skin test reactions to tuberculin are associated with increasing rates of subsequent progression to active tuberculosis. However, the prognostic reliability of the TST in vulnerable populations, such as young children, may be reduced. The application of the skin test also has logistical limitations; for example, the need for a return visit 3 to 7 days later to read the result and the subjectivity in recording the amount of cutaneous induration.

### Summary of the Invention

The present inventors have shown that T cell responses to particular mycobacterial antigens act as prognostic markers that can be used to identify individuals at risk of progressing to active mycobacterial disease. This finding is used as the basis of the prognostic method of the invention.

Accordingly, the present invention provides a method for detecting whether an individual will progress to having active mycobacterial disease comprising determining whether the individual has a T cell response to one or more of the following mycobacterial antigens:
- CFP-10,
- Rv3873, or
- Rv3878.

The invention also provides the use in the prognostic method of the invention of a list comprising means for determining whether the individual has a T cell response to one or more of the following mycobacterial antigens:
- CFP-10,
- Rv3873 or
- Rv3878.

### Brief Description of the Drawing

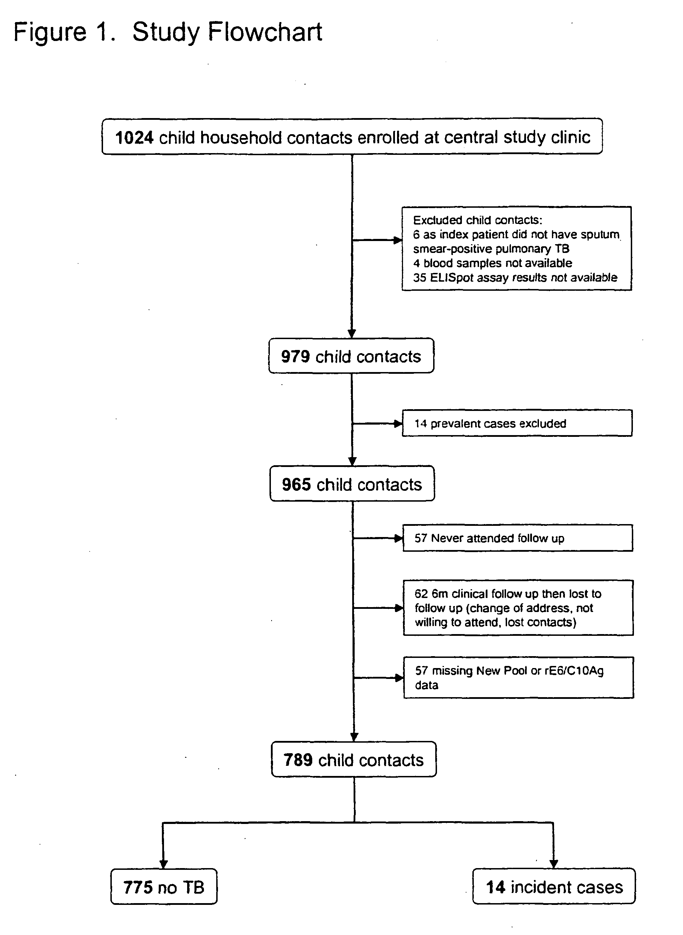

Figure 1 shows a flowchart for the study described in the Examples.

### Brief Description of the Sequences

SEQ ID NO: 1 is the amino acid sequence of the *Mycobacterium tuberculosis* antigen ESAT-6.
SEQ ID NO: 2 is the amino acid sequence of the *Mycobacterium tuberculosis* antigen CPF10.
SEQ ID NO: 3 is the amino acid sequence of the *Mycobacterium tuberculosis* antigen Rv1989c.
SEQ ID NO: 4 is the amino acid sequence of the *Mycobacterium tuberculosis* antigen Rv3873.
SEQ ID NO: 5 is the amino acid sequence of the *Mycobacterium tuberculosis* antigen Rv3878.
SEQ ID NO: 6 is the amino acid sequence of the *Mycobacterium tuberculosis* antigen Rv3879c.
ES1 to ES17 are the amino acids sequences of a pool of 17 peptides derived from ESAT-6.
cfp10/1 to cfp10/18 are the amino acids sequences of a pool of 18 peptides derived from CFP-10.
NEW POOL 1 lists the amino acids sequences of a pool of 6 peptides derived from Rv3873.
NEW POOL 3 lists the amino acids sequences of a pool of 6 peptides derived from Rv3873.
NEW POOL 4 lists the amino acids sequences of a pool of 7 peptides derived from Rv3878.
NEW POOL 5 lists the amino acids sequences of a pool of 7 peptides derived from Rv3878.
NEW POOL 6 lists the amino acids sequences of a pool of 6 peptides derived from Rv3879c.
NEW POOL 7 lists the amino acids sequences of a pool of 6 peptides derived from Rv3879c.
NEW POOL 8 lists the amino acids sequences of a pool of 5 peptides derived from Rv3879c.
NEW POOL 9 lists the amino acids sequences of a pool of 6 peptides derived from Rv1989c.
NEW POOL 10 lists the amino acids sequences of a pool of 6 peptides derived from Rv1989c.
NEW POOL 11 lists the amino acids sequences of a pool of 6 peptides derived from Rv1989c.

### Detailed Description of the Invention

The invention provides a method of detecting whether an individual will progress to having active mycobacterial disease. In a preferred embodiment such a mycobacterial disease is caused by *Mycobacterium tuberculosis.*

The individual is typically a mammal, preferably a human. The individual is typically one that does not have any symptoms of a mycobacterial infection. The individual may test positive or negative in a Mantoux test. The individual may be at risk of mycobacterial infection, typically for socio-economic reasons or may have a genetic or acquired predisposition to mycobacterial infection.

The individual may be a known or suspected "contact" who has been exposed to or may have been exposed to *Mycobacterium tuberculosis.* Typically the exposure is to pulmonary tuberculosis, such as "open" pulmonary tuberculosis which is sputum A.F.B. (acid-fast bacillus) smear positive. The contact may be someone whose exposure is a household, work place (such as a health care worker) or prison exposure (such as a prisoner). The exposure may have resulted from residing in a country with high prevalence of TB, and in one embodiment the prognostic method is carried out after emigration to a country with a low prevalence of TB. Thus the individual may be an immigrant.

The individual (for example who has a known or suspected recent or remote exposure) may be healthy, might have a co-infection or a chronic condition or be on therapeutic agents putting them at a higher risk of developing active TB and/or which may make TB infection harder to diagnose. Examples include HIV infected individuals, individuals taking immunosuppressants (e.g. corticosteroids, azathioprine and anti-TNF-α agents, such as infliximab, and cancer therapy), hemodialysis patients, organ transplant recipients, diabetics and very young children (aged under 5 years old, particularly under 2 years old).

The method of the invention concerns determining whether an individual has a T cell response to particular antigens. This may be determined by any suitable method, for example any method which can be used to detect the presence of antigen-experienced T cells. In one embodiment it is determined by measuring the level of T cells specific to the antigen(s). It can be determined by determining whether T cells of the individual recognise the antigen(s).

The T cells which recognise the antigen in the method are generally T cells which have been pre-sensitised *in vivo* to antigen from a mycobacterium. These antigen-experienced T cells are generally present in the peripheral blood of a individual which has been exposed to a mycobacterium at a frequency of 1 in 10⁶ to 1 in 10³ peripheral blood mononuclear cells (PBMCs). The T cells may be CD4 and/or CD8 T cells.

In the method the T cells can be contacted with the antigen *in vitro,* preferably *in vitro* in a sample from the individual.

Generally the T cells which are contacted in the method are taken from the individual in a blood sample, although other types of samples which contain T cells can be used. The sample may be added directly to the assay or may be processed first. Typically the processing may comprise diluting of the sample, for example with water, buffer or media. Typically the sample is diluted from 1.5 to 100 fold, for example 2 to 50 or 5 to 10 fold.

The processing may comprise separation of components of the sample. Typically mononuclear cells (MCs) are separated from the samples. The MCs will comprise the T cells and antigen presenting cells (APCs). Thus in the method the APCs present in the separated MCs can present the peptide to the T cells. In another embodiment only T cells, such as only CD4 T cells, can be purified from the sample. PBMCs, MCs and T cells can be separated from the sample using techniques known in the art, such as those described in Lalvani et al (1997) J. Exp. Med. 186, p859-865.

Preferably the T cells used in the assay are in the form of unprocessed or diluted samples, are freshly isolated T cells (such as in the form of freshly isolated MCs or PBMCs) which are used directly *ex vivo,* i.e. they are not cultured before being used in the method or are thawed cells (which were previously frozen). However the T cells can be cultured before use, for example in the presence of the antigen, and generally also exogenous growth promoting cytokines. During culturing the antigen is typically present on the surface of APCs, such as the APC used in the method. Pre-culturing of the T cells may lead to an increase in the sensitivity of the method. Thus the T cells can be converted into cell lines, such as short term cell lines (for example as described in Ota et al (1990) Nature 346, p183-187).

The APC which is typically present in the method may come from the same individual as the T cell or from a different individual. The APC may be a naturally occurring APC or an artificial APC. The APC is a cell which is capable of presenting the antigen to a T cell. It is typically a B-cell, dendritic cell or macrophage. It is typically separated from the same sample as the T cell and is typically co-purified with the T cell. Thus the APC may be present in MCs or PBMCs. The APC is typically a freshly isolated *ex vivo* cell or a cultured cell. It may be in the form of a cell line, such as a short term or immortalised cell line. The APC may express empty MHC class II molecules on its surface.

In one embodiment the antigen is added directly to an assay comprising T cells and APCs. As discussed above the T cells and APCs in such an assay could be in the form of MCs. When an antigen which can be recognised by the T cell without the need for presentation by APCs then APCs are not required. Analogues which mimic the original antigen bound to a MHC molecule are an example of such an antigen.

In one embodiment the antigen is provided to the APC in the absence of the T cell. The APC is then provided to the T cell, typically after being allowed to present the antigen on its surface. The antigen may have been taken up inside the APC and presented, or simply be taken up onto the surface without entering inside the APC.

Typically 10⁵ to 10⁷, preferably 2.5x10⁵ to 10⁶ PBMCs are added to each assay. In the case where peptide is added directly to the assay its concentration is from 10⁻¹ to 10³µg/ml, preferably 0.5 to 50µg/ml or 1 to 10µg/ml.

Typically the length of time for which the T cells are incubated with the antigen is from 4 to 24 hours (preferably 5 to 18 hours) for effector T cells or for more than 24 hours for central memory cells. When using *ex vivo* PBMCs it has been found that 5.0 x10⁶ PBMCs can be incubated in 10µg/ml of peptide for 5 hours at 37°C.

The antigen may be in any suitable form. The antigen generally comprises one or more T cell epitopes from CFP-10, Rv3873 or Rv3878. Thus in one embodiment peptides are used which are fragments of CFP-10, Rv3873 or Rv3878, although the whole form (as shown in SEQ ID NO's 2 to 5) of any of these proteins is preferably used. In addition peptides can be used which comprise sequences which are recognised by the T cells which recognise epitopes in CFP-10, Rv3873 or Rv3878. Such sequences may have at least 70%, 80%, 90% homology to the original epitope sequence.

The peptides used in the invention typically have a length of from 8 to 1000 amino acids, such as from 10 to 500, 15 to 200 or 20 to 100 amino acids.

In one embodiment T cell responses to 2 or more of CFP-10, Rv3873 or Rv3878 are investigated, for example using combinations of the peptides. In this embodiment T cell responses to ESAT-6 may also be investigated to increase the power of the prognostic method of the invention. Again, the peptides used may comprise a fragment of ESAT-6 or, preferably, the whole form (as shown in SEQ ID NO 1), and/or homologous sequences recognised by the T cells which recognise epitopes in ESAT-6. In a particularly preferred embodiment, the T cell responses to the combination of ESAT-6 and CFP-10 are investigated.

The antigen may be a fragment (such as a peptide) and/or homologue of a naturally occurring protein which is recognised by a T cell that recognises the natural T cell epitope sequence.

A cytokine (such as IL-2 or IFN-γ) can typically be detected by allowing them to bind to a specific capture agent which may be immobilised on a support such as a plate,

A cytokine (such as IL-2 or IFN-γ) can typically be detected by allowing them to bind to a specific capture agent which may be immobilised on a support such as a plate, bead or the cytokine-secreting cell itself, and then measuring the presence of the specific binding agent/cytokine complex typically with a second binding detection agent. A washing step can be incorporated to remove material which is not specifically bound to the capture agent.

Typically the second agent binds the cytokine at a site which is different from the site which binds the first agent. The second agent may be directly conjugated to an enzyme such as alkaline phosphatase, or fluorescent label or may comprise a biotin moiety to be detected by a third agent comprising streptavidin, which is directly conjugated to an enzyme or fluorescent label. The conjugated enzyme then changes colour of a reagent. The agent is preferably an antibody, mono- or polyclonal. Antibodies to cytokines are commercially available, or can be made using standard techniques.

The preferred method employed to detect cytokines will be based on sandwich immunoassays detecting the frequency of cytokine-secreting cells such as colour or fluorescent ELISpot, limited dilution assays, intracellular cytokine staining and cytokine secretion assays with or without enrichment of cytokine-secreting cells as pioneered by Miltenyi Biotec. Alternatively, the amount of cytokine secreted can be measured for example by an ELISA based system such as the whole blood Quantiferon® system with the capture antibody immobilised on a plate, and its modifications (for example as available from Cellestis) or Luminex® suspension array technology using Beadlyte® kits with the capture antibody immobilised on a bead. Cytokine mRNA expression can also be measured with assays such as RT-PCR.

In one embodiment the detection system which is used is the *ex-vivo* ELISpot assay described in WO 98/23960. In that assay IFN-γ secreted from the T cell is bound by a first IFN-γ specific antibody which is immobilised on a solid support. The bound IFN-γ is then detected using a second IFN-γ specific antibody which is labelled with a detectable label. Such a labelled antibody can be obtained from MABTECH (Stockholm, Sweden). Other detectable labels which can be used are discussed below.

### Kits

The invention also provides the use of a kit for carrying out the methods of the invention comprising a means for determining whether an individual has a T cell response to any of CFP-10, Rv3873 or Rv3878. Preferably the kitfurther comprises peptides from one or more of CFP-10, Rv3873, and/or Rv3878, optionally also peptides from ESAT-6 and optionally antibodies to IFN- y or IL-2 immobilised on a solid support. Typically the means to detect recognition allows or aids detection based on the techniques discussed above. Thus, the means may allow detection of cytokine (such as IFN-γ and/or IL-2) secreted by the T cells after recognition. The kit may thus additionally include a specific binding agent for the cytokine, such as an antibody. The agent is typically immobilised on a solid support. This means that after binding the agent the cytokine will remain in the vicinity of the T cell that secreted it. Thus 'spots' of cytokine/agent complex are formed on the support, each spot representing a T cell which is secreting the cytokine. Quantifying the spots, and typically comparing against a control, allows determination of the relative numbers of cells that secrete the cytokine.

The kit may also comprise a means to detect the cytokine/agent complexes. A detectable change may occur in the agent itself after binding cytokine, such as a colour change. Alternatively a second agent directly or indirectly labelled for detection may be allowed to bind the cytokine /agent complex to allow the determination of the spots. As discussed above the second agent may be specific for the cytokine, but binds a different site on the cytokine than the first agent.

The immobilised support may be a plate with wells, such as a microtitre plate. Each assay can therefore be carried out in a separate well in the plate.

The kit may additionally comprise medium for the T cells, detection agents or washing buffers to be used in the detection steps. The kit may additionally comprise reagents suitable for the separation from the sample, such as the separation of PBMCs or T cells from the sample. The kit may be designed to allow detection of the T cells directly in the sample without requiring any separation of the components of the sample.

The kit may also comprise controls, such as positive or negative controls. The positive control may allow the detection system to be tested. Thus the positive control typically mimics recognition of the peptide in any of the above methods. Typically in the kits designed to determine recognition *in vitro* the positive control is a cytokine, such as IFN-γ and/or IL-2.

The kit may also comprise a means to take a sample containing T cells from the human, such as a blood sample. The kit may comprise a means to separate mononuclear cells or T cells from a sample from the individual.

### Therapy

In the event that an individual is found, by means of the method of the present invention, to be at risk of progressing to mycobacterial disease the individual may be treated by administering an agent which is therapeutic for a mycobacterial infection or mycobacterial disease.

The individual may be given an agent which treats a mycobacterial infection and/or mycobacterial disease. The agent may be a natural agent such as a vitamin (e.g. vitamin D), mineral or plant-derived product. The agent may be a preventative or therapeutic vaccine. The agent is typically isoniazid, rifampicin, ethambutol, pyrazinamide, streptomycin, para-amino-salicyclic acid, kanamyin, capreomycin, ethionamide, cycloserine, thiacetazone or a flouroquinolone (e.g. ciprofloxacin).

The individual (who is in need of treatment) is typically given an effective nontoxic amount of the agent. The agent may be in the form of a pharmaceutical composition which comprises the agent and a pharmaceutically acceptable carrier or diluent. Suitable carriers and diluents include isotonic saline solutions, for example phosphate-buffered saline. Typically the product is administered by parenteral, intravenous, intramuscular, subcutaneous, transdermal, intradermal, oral, intranasal, inhalation (into the lungs), intravaginal, or intrarectal administration.

The dose of the product may be determined according to various parameters, especially according to the particular agent; the age, weight and condition of the patient to be treated; the route of administration; and the required regimen. A physician will be able to determine the required route of administration and dosage for any particular patient. A suitable dose may however be from 10µg to 10g, for example from 100 µg to 1g of the product.

### Homology

As mentioned above a homologue of a mycobacterial sequence may be used in the method of the invention. A peptide which is homologous to another peptide is typically at least 70% homologous to the peptide, preferably at least 80 or 90% and more preferably at least 95%. 97% or 99% homologous thereto, for example over a region of at least 8. preferably at least 15, for instance at least 40, 60 or 100 or more contiguous amino acids, or over its entire length. The homologue typically differs from the protein or peptide by 1,2, less than 6, such as less than 12 mutations (each of which is a substitution (e.g. a conservative substitution), deletion or insertion) for example over any of the above-mentioned lengths of region mentioned for homology.

Methods of measuring protein homology are well known in the art and it will be understood by those of skill in the art that in the present context, homology is calculated on the basis of amino acid identity (sometimes referred to as "hard homology"). For example the UWGCG Package provides the BESTFIT program which can be used to calculate homology (for example used on its default settings) (Devereux et al (1984) Nucleic Acids Research 12, p387-395).

In the homologue, amino acids which are equivalent to amino acids in the original protein or peptide which contribute to binding the MHC molecule or are responsible for the recognition by the T cell receptor, may be the same or may be conservatively substituted. Conservative substitutions are defined in the table below. Amino acids in the same block in the second column and preferably in the same line in the third column may be substituted for each other:

| | | |
|---|---|---|
| ALIPHATIC | Non-polar | G A P |
| | | IL V |
| | Polar-uncharged | C S T M |
| | | N Q |
| | Polar-charged | D E |
| | | KR |
| AROMATIC | | H F W Y |

The following Examples illustrate the invention.

### Examples

### Study Participants

All adults diagnosed with sputum smear-positive pulmonary tuberculosis at any of the 7 government-funded tuberculosis clinics on the Asian side of Istanbul between October 2002 and May 2004 were asked if they had children living in the household and were invited to participate in the study. Four hundred and forty three patients had one or more child household contacts and all agreed to participate and gave written informed consent on behalf of their children. Where the index case was not the parent, consent was given by the child's parents or legal guardian. Contacts were included if they were 16 years old or younger, and prevalent cases of active tuberculosis were excluded.

The Turkish Ministry of Health guidelines for BCG vaccination are as follows: all children are vaccinated intradermally with BCG Pasteur 1173-P2 (Serum Institute of India Ltd., Pune, India) between 2 and 3 months of age and a booster vaccination is administered in the first year of primary school, at 6 to 7 years of age. Surveillance data for BCG vaccination coverage in Turkey indicate a vaccination rate of 79% in children in 2004.

### Clinical Evaluation and Treatment

All 1,024 child contacts of the 443 index patients with sputum smear positive pulmonary tuberculosis were enrolled on the same two weekdays every week at the Paediatric Infectious Diseases Clinic, Marmara University School of Medicine between October 2002 and May 2004. A full medical history, physical examination and documentation of BCG vaccination scars were carried out by the study paediatricians. Demographic and socioeconomic information was recorded by the study nurse. All children received a TST and chest radiography and 1,020 had a 10 ml venous blood sample taken for the ELISpot assay.

Children with clinical features or radiographic findings suggestive of active tuberculosis were further investigated as directed by the clinical presentation. Where a final diagnosis of active tuberculosis was made, children were treated with 6 months standard short course chemotherapy and followed up for one year.

A 6 month course of isoniazid preventive therapy was given in accordance with Turkish Ministry of Health guidelines. All contacts were followed up 6 monthly at the clinic but were asked to return immediately for further clinical assessment if they developed intercurrent symptoms consistent with tuberculosis.

### Antigens

CFP10, Rv3873 and Rv3878 are all encoded by Region of Difference 1 (RDI) genomic segment. Rv1989c is encoded in RD2. RD1 and RD2 are absent from most strains of *M*. *bovis BCG* vaccine and most environmental mycobacteria, but are present in all strains of *M*. *tuberculosis.*

### Tuberculin Skin Test

TST was administered to all children by the Mantoux method using 0·1 ml (2 tuberculin units) of purified protein derivative (PPD) RT23 (Statens Serum Institut, Copenhagen, Denmark). The test was performed and read by the study paediatrician who was blinded to ELISpot results. The cutaneous appearance of peau d'orange was noted in all participants, confirming intradermal inoculation of PPD. Induration was measured after 48-72 hours with a ruler. TST response was scored as positive if induration diameter was ≥10 millimetres in contacts not BCG vaccinated and induration ≥15 millimetres in BCG vaccinated contacts. Contacts with a negative TST result had a repeat TST 2 to 6 months later. TST conversion was defined as an increase of ≥6 mm and the second TST induration to be ≥10 mm if BCG unvaccinated and ≥15 mm if BCG vaccinated.

### Ex-vivo Interferon-γ ELISpot

A 10 mL venous blood sample was taken for the ELISpot assay. Briefly, precoated interferon-y ELISpot plates (Mabtech AB, Stockholm, Sweden) were seeded with 2·5 x 10⁵ peripheral blood mononuclear cells per well: duplicate wells contained no antigen (negative control), phytohemagglutinin (positive control; ICN Biomedical, OH, USA) at 5 µg/mL, streptokinase-streptodornase (non-MTB related antigen) at 20.8 IU/mL and a further 19 pairs of duplicate wells, each containing recombinant ESAT-6 antigen (SEQ ID NO:1) at 8.34 µg/mL, recombinant CFP10 antigen (SEQ ID NO:2) at 8.34 µg/mL, purified protein derivative (PPD) at 16.7 µg/mL and 1 of 16 peptide pools which incorporated 5, 6 or 7 15mer peptides from 96 such peptides spanning the length of ESAT-6 or CFP10, or selected regions from Rv3873, Rv3878, Rv3879c and Rv1989c such that the final concentration of each peptide was 10 µg/mL. Previously described non-specific peptides from Rv3873 were excluded. After overnight incubation at 37°C in 5% CO₂, the plates were developed with preconjugated detector antibody (Mabtech AB) followed by chromogenic substrate (Moss Inc., Pasadena, MD, USA). ELISpot plates were scored in Oxford by an automated ELISpot counter (AID-GmbH, Strassberg, Germany). Intensity and spot size settings were pre-defined and the same settings were used throughout. Mean readings from duplicate wells were electronically transferred to a spreadsheet by a customised software programme, ELISTAT (AID-GmbH, Strassberg, Germany). Responses were scored as positive if the test wells contained a mean of at least 5 spot-forming cells more than the mean of the negative control wells, and, in addition, this number was at least twice the mean of the negative control wells. Persons performing and reading the assays were blind to all personal identifiers and TST results.

*Ex-vivo* IFN-γ ELISpot assays were repeated 6 months post recruitment, including the negative and positive controls as described above. However, the repeat assays only included a further 6 pairs of duplicate wells, each containing 1 of 6 pools which incorporated 5 or 6 15mer peptides from 35 such peptides spanning the length of ESAT-6 or CFP10.

### Incident case definitions

Child contacts were clinically followed up every 6 months for 2 years at the clinic but asked to return immediately for further clinical assessment if they developed intercurrent symptoms. Diagnosis of active tuberculosis was made by the study paediatricians, taking into account symptoms, physical signs, and radiological and microbiological findings. Children diagnosed with active pulmonary tuberculosis were treated with 6 months standard short-course chemotherapy. Children diagnosed with miliary tuberculosis were treated with isoniazid, rifampicin, streptomycin and pyrazinamide for 2 months followed by isoniazid and rifampicin for a further 10 months. Children diagnosed with multidrug resistant tuberculosis were treated with second line agents based on antibiotic susceptibility profiles.

### Statistical analysis

Differences between the proportions of contacts responding to each particular antigen were compared using the McNemar's test for paired binary variables. The strength of T-cell responses to particular antigens amongst responders were compared using the Mann-Whitney test for continuous variables. Relative risk and 95% confidence intervals (95% CI), their significance being assessed by P-values calculated by the Chi squared test, were used to assess the prognostic value of the assay result for TST conversion, ESAT-6/CFP10 ELISpot conversion and progression to active TB disease. All analyses were undertaken in Graphpad Prism 4 for Windows (Version 4.03, GraphPad Software, Inc., CA, USA) and SPSS for Windows (Rel 13.0, SPSS Inc, Chicago, USA).

### Conclusions

Prognostic markers can help to identify patients at different degrees of risk for specific outcomes and facilitate treatment choice. We assessed the prognostic value of a positive ex vivo IFN-γ ELISpot response to the antigens rESAT-6 (SEQ ID NO:1), rCFP-10 (SEQ ID NO:2), and to peptides spanning the length of these antigens (peptides ES1 to ES17 for ESAT-6 and peptides cf10/1 to cfp10/18 for CFP-10), and to peptides derived from Rv3873 (NEW POOL 1 and NEW POOL 3), RV3878 (NEW POOL 4 and NEW POOL 5) and Rv1989c (NEW POOL 9, 10 & 11) in predicting progression to active tuberculosis in 789 child household contacts of adults with sputum smear-positive pulmonary tuberculosis. A positive ELISpot response to ESAT-6, CFP-10, Rv3873 or RV3878 was prognostic of progression to active tuberculosis over two years (table 1). The relative incident rate was similar in child contacts ELISpot-positive to ESAT-6, CFP-10, Rv3873 and Rv3878 peptides (table 1). Children ELISpot-positive to rESAT-6 or rCFP-10 had a higher incident rate (table 1).
ELISpot responses to PPD, a mixture of around 200 *M*. *tb* antigens, or to SKSD, an antigen not found in *M*. *tb,* were not prognostic of subsequent active tuberculosis (table 1). Using more than one antigen or peptides from different antigens in specific combinations in the ELISpot assay identified more children at risk of progression to active tuberculosis (table 2). Notably, not all responses to *M. tb* antigens confer increased risk of progression to active tuberculosis, as evidenced by the lack of prognostic value of T cell responses to Rv3879c (NEW POOLS 6, 7 and 8, table 3) and PPD (table 1), as previously mentioned. Detecting T cell responses to mycobacterial antigens or peptides would improve identification of contacts most likely to progress to active tuberculosis and help clinicians to estimate accurately the risk of progression to disease in guidance for the decision of initiation of preventive therapy.

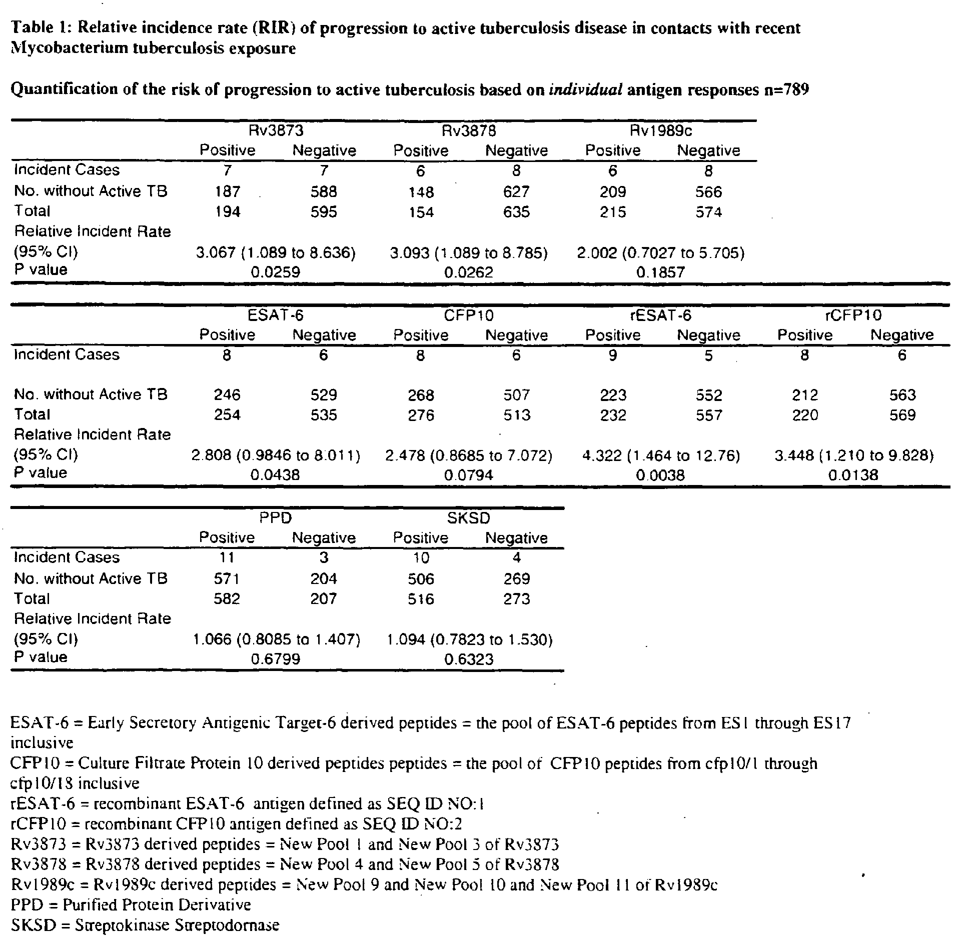

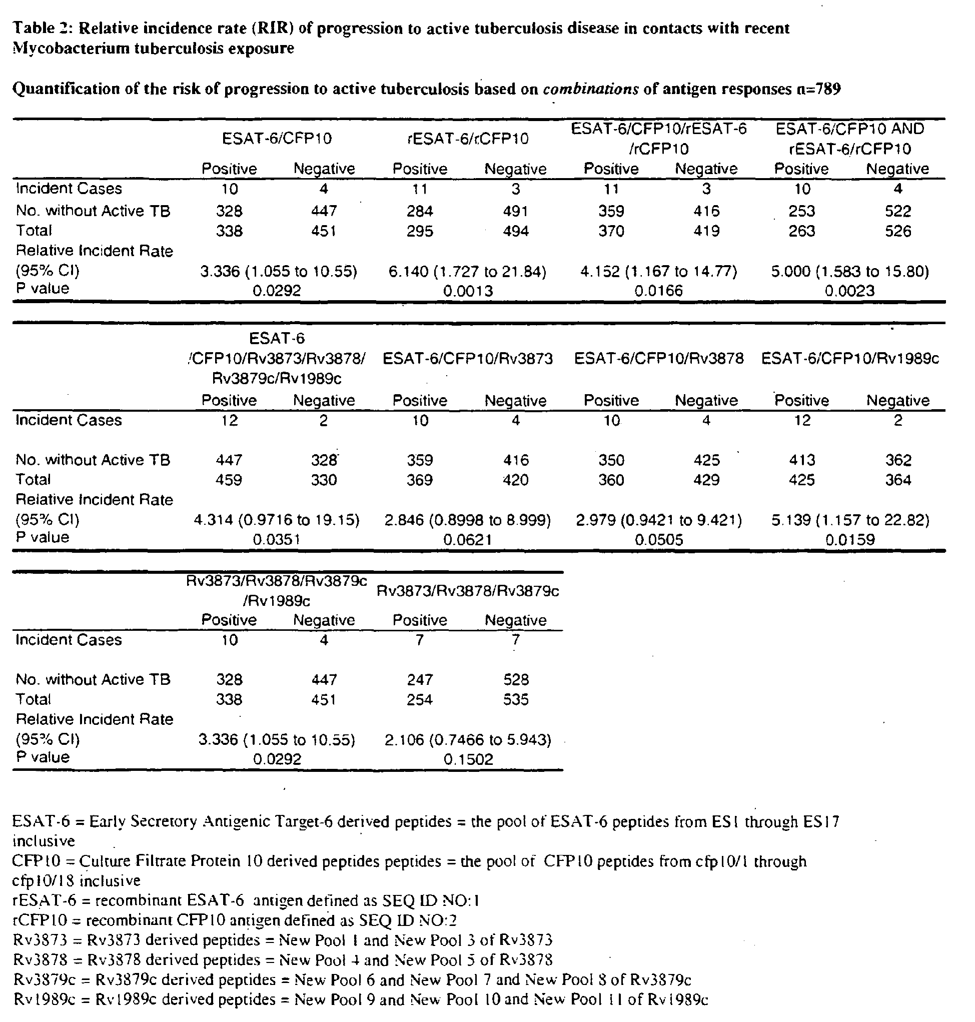

| Rv3873/Rv3878/Rv3879c/Rv1989c/rESAT-6/rCFP10 | | Rv3873/Rv3878/Rv3879c/rESAT-6/rCFP10 | | Rv1989c/rESAT-6/rCFP10 | |
|---|---|---|---|---|---|
| Positive | Negative | Positive | Negative | Positive | Negative |
| 12 | 2 | 11 | 3 | 12 | 2 |
| 432 | 343 | 370 | 405 | 384 | 391 |
| 444 | 345 | 381 | 408 | 396 | 393 |
| 4.662 (1.050 to 20.70) | | 3.927 (1.104 to 13.97) | | 5.955 (1.341 to 26.44) | |
| 0.0250 | | 0.0221 | | 0.0073 | |

The table above shows use of combinations of peptides. Again the columns show incident cases, number
without active TB, total, relative incident rate (95% CI) and P value

**Table 3. Negative Result for Rv3879c**

| | Rv3879c | |
|---|---|---|
| | Positive | Negative |
| Incident Cases | 5 | 9 |
| No. without Active TB | 186 | 589 |
| Total | 191 | 598 |
| Relative Incident Rate (95% CI) | 1.739 (0.5899 to 5.128) | |
| P value | 0.3105 | |

| | | |
|---|---|---|
| Rv3879c = Rv3879c derived peptides = New Pool 6 and New Pool 7 and New Pool 8 of Rv3879c | | |

Rv 3873 (SEQ ID NO:4) 
1 mlwhamppel ntarlmagag papmlaaaag wqtlsaalda qaveltarln slgeawtggg
61 sdkalaaatp mvvwlqtast qaktramqat aqaaaytqam attpslpeia anhitqavlt
121 atnffginti pialtemdyf irmwnqaala mevyqaetav ntlfeklepm asildpgasq
181 sttnpifgmp spgsstpvgq lppaatqtlg qlgemsgpmq qltqplqqvt slfsqvggtg
241 ggnpadeeaa qmgllgtspl snhplaggsg psagagllra eslpgaggsl trtplmsqli
301 ekpvapsvmp aaaagssatg gaapvgagam gqgaqsggst rpglvapapl aqereedded
361 dwdeeddw
NEW POOL1
   1. ATAQA AAYTQ AMATT
   2. AAYTQ AMATT PSLPE
   3. AMATT PSLPE IAANH
   4. PSLPE IAANH ITQAV
   5. IAANH ITQAV LTATN
   6. ITQAV LTATN FFGIN
NEW POOL3
   7. LAMEV YQAET AVNTL
   8. YQAET AVNTL FEKLE
   9. AVNTL FEKLE PMASI
   10. FEKLE PMASI LDPGA
   11. PMASI LDPGA SQSTT
   12. LDPGA SQSTT NPIFG
RV3878 (SEQ ID NO:5) 
1 maeplavdpt glsaaaakla glvfpqppap iavsgtdsvv aainetmpsi eslvsdglpg
61 vkaaltrtas nmnaaadvya ktdqslgtsl sqyafgssge glagvasvgg qpsqatqlls
121 tpvsqvttql getaaelapr vvatvpqlvq laphavqmsq naspiaqtis qtaqqaaqsa
181 qggsgpmpaq lasaekpate qaepvhevtn ddqgdqgdvq paevvaaard egagaspgqq
241 pgggvpaqam dtgagarpaa splaapvdps tpapstttl
NEW POOL4
   1. MAEPL AVDPT GLSAA
   2. AVDPT GLSAA AAKLA
   3. GLSAA AAKLA GLVFP
   4. AAKLA GLVFP QPPAP
   5. GLVFP QPPAP IAVSG
   6. QPPAP IAVSG TDSVV
   7. IAVSG TDSVV AAINE
NEW POOL5
   8. TDSVV AAINE TMPSI
   9. AAINE TMPSI ESLVS
   10. TMPSI ESLVS DGLPG
   11. ESLVS DGLPG VKAAL
   12. DGLPG VKAAL TRTAS
   13. VKAAL TRTAS NMNAA
   14. TRTAS NMNAA ADVYA
Rv1989c (SEQ ID NO:3) 
1   msdaldeglv qridargtie wsetcyrytg ahrdalsgeg arrfggrwmp pllfpaiyla
61  dsaqacmvev eraaqaastt aekmleaayr lhtidvtdla vldlttpqar eavglenddi
121 ygddwsgcqa vghaawflhm qgvlvpaagg vglvvtayeq rtrpgqlqlr qsvdltpaly
131 qelrat
NEW POOL 9
   1. VSDAL DEGLV QRIDA
   2. DEGLV QRIDA RGTIE
   3. QRIDA RGTIE WSETC
   4. RGTIE WSETC YRYTG
   5. WSETC YRYTG AHRDA
   6. YRYTG AHRDA LSGEG
NEW POOL10
   7. AHRDA LSGEG ARRFG
   8. LSGEG ARRFG GRWNP
   9. ARRFG GRWNP PLLFP
   10. GRWNP PLLFP AIYLA
   11. PLLFP AIYLA DSAQA
   12. AIYLA DSAQA CMVEV
NEW POOL11
   13. DSAQA CMVEV ERAAQ
   14. CMVEV ERAAQ AASTT
   15. ERAAQ AASTT AEKML
   16. AASTT AEKML EAAYR
   17. AEKML EAAYR LHTID
   18. EAAYR LHTID VTDLA
CFP-10 (SEQ ID NO:2) 
MAEMK TDAAT LAQEA GNFER ISGDL KTQID QVEST AGSLQ GQWRG GQWRG AAGTA
AQAAV VRFQE AANKQ KQELD EISTN IRQAG VQYSR ADEEQ QQALS SQMGF
cfp10/1 MAEMK TDAAT LAQEA
cfp10/2 TDAAT LAQEA GNFER
cfp10/3 LAQEA GNFER ISGDL
cfp10/4 GNFER ISGDL KTQID
cfp10/5 ISGDL KTQID QVEST
cfp10/6 KTQID QVEST AGSLQ
cfp10/7 QVEST AGSLQ GQWRG
cfp10/8 AGSLQ GQWRG AAGTA
cfp10/9 GQWRG AAGTA AQAAV
cfp10/10 AAGTA AQAAV VRFQE
cfp10/11 AQAAV VRFQE AANKQ
cfp10/12 VRFQE AANKQ KQELD
cfp10/13 AANKQ KQELD EISTN
cfp10/14 KQELD EISTN IRQAG
cfp10/15 EISTN IRQAG VQYSR
cfp10/16 IRQAG VQYSR ADEEQ
cfp10/17 VQYSR ADEEQ QQALS
cfp10/18 ADEEQ QQALS SQMGF
ESAT-6 (SEQ ID NO:1) 
MTEQQ WNFAG IEAAA SAIQG NVTSI HSLLD EGKQS LTKLA AAWGG SGSEA YQGVQ
QKWDA TATEL NNALQ NLART ISEAG QAMAS TEGNV TGMFA
ES1: MTEQQ WNFAG IEAAA
ES2: WNFAG IEAAA SAIQG
ES3: IEAAA SAIQG NVTSI
ES4: SAIQG NVTSI HSLLD
ESS: NVTSI HSLLD EGKQS
ES6: HSLLD EGKQS LTKLA
ES7: EGKQS LTKLA AAWGG
ES8: LTKLA AAWGG SGSEA
ES9: AAWGG SGSEA YQGVQ
ES10: SGSEA YQGVQ QKWDA
ES11: YQGVQ QKWDA TATEL
ES12: QKWDA TATEL NNALQ
ES13: TATEL NNALQ NLART
ES14: NNALQ NLART ISEAG
ES15: NLART ISEAG QAMAS
ES16: ISEAG QAMAS TEGNV
ES17: QAMAS TEGNV TGMFA
SEQ ID NO:6 - Rv3879c 
1 msitrptgsy arqmldpggw veadedtfyd raqeysqvlq rvtdvldtcr qqkghvfegg
61 lwsggaanaa ngalganinq lmtlqdylat vitwhrhiag lieqaksdig nnvdgaqrei
121 dilendpsld aderhtains lvtathganv slvaetaerv lesknwkppk naledllqqk
181 sppppdvptl vvpspgtpgt pgtpitpgtp itpgtpitpi pgapvtpitp tpgtpvtpvt
241 pgkpvtpvtp vkpgtpgept pitpvtppva patpatpatp vtpapaphpq papapapspg
301 pqpvtpatpg psgpatpgtp ggepaphvkp aalaeqpgvp gqhagggtqs gpahadesaa
361 svtpaaasgv pgaraaaaap sgtavgagar ssvgtaaasg agshaatgra pvatsdkaaa
421 pstraasart apparppstd hidkpdrses addgtpvsmi pvsaaraard aataaasarq
481 rgrgdalrla rriaaalnas dnnagdygff witavttdgs ivvansygla yipdgmelpn
541 kvylasadha ipvdeiarca typvlavqaw aafhdmtlra vigtaeqlas sdpgvakivl
601 epddipesgk mtgrsrlevv dpsaaaqlad ttdqrlldll ppapvdvnpp gderhmlwfe
661 lmkpmtstat greaahlraf rayaahsqei alhqahtatd aavqrvavad wlywqyvtgl
721 ldralaaac
NEW POOL6
   1. MSITR PTGSY ARQML
   2. PTGSY ARQML DPGGW
   3. ARQML DPGGW VEADE
   4. DPGGW VEADE DTFYD
   5. VEADE DTFYD RAQEY
   6. DTFYD RAQEY SQVLQ
NEW POOL7
   7. RAQEY SQVLQ RVTDV
   8. SQVLQ RVTDV LDTCR
   9. RVTDV LDTCR QQKGH
   10. LDTCR QQKGH VFEGG
   11. QQKGH VFEGG LWSGG
   12. VFEGG LWSGG AANAA
NEW POOL8
   13. LWSGG AANAA NGALG
   14. AANAA NGALG ANINQ
   15. NGALG ANINQ LMTLQ
   16. ANINQ LMTLQ DYLAT
   17. LMTLQ DYLAT VITWH

## Claims

1. A method for detecting whether an individual will progress to having active mycobacterial disease comprising determining whether the individual has a T cell response to one or more of the following mycobacterial antigens:
- CFP-10,
- Rv3873, or
- Rv3878.

2. A method according to claim 1 wherein the determination of whether the individual has a T cell response to the mycobacterial antigen is carried out by assessing:
- whether T cells of the individual recognise said mycobacterial antigen, and/or
- the level ofT cells that are specific to said mycobacterial antigen in a sample from the individual.

3. A method according to claim 1 or 2 comprising determining whether the individual has a T cell response to 2, 3, 4 or all of the following: ESAT-6, CFP-10, Rv3873 or Rv3878.

4. A method according to claim 3 comprising determining whether the individual has a T cell response to ESAT-6 and CFP-10.

5. A method according to any one of the preceding claims wherein T cells of the individual are contacted with a peptide that comprises a T cell epitope of ESAT-6, CFP-10, Rv3873 or Rv3878; and the response of the T cells to the peptide is assessed.

6. A method according to any one of the preceding claims comprising determination of T-cell recognition of a peptide comprising SEQ ID NO:1, SEQ ID NO:2, SEQ ID NO:4, SEQ ID NO:5, or SEQ ID NO:6, or a homologue of one of said sequences which is at least 70% homologous thereto.

7. A method according to any one of claims 1 to 5 comprising determination of T cell recognition of a fragment and/or homologue of ESAT-6, CFP-10, Rv3873 or Rv3878, wherein said fragment and/or homologue is capable of being recognised by a T cell that recognises a T cell epitope of ESAT-6, CFP-10, Rv3873 or Rv3878.

8. A method according to any one of the preceding claims wherein the T cells are present in a sample from the individual, and
- all of the peptides that are to be tested are provided simultaneously to the sample, and/or
- less than 20, and preferably less than 10, different peptides are provided to the sample, and/or
- at least 1 or 2 peptides from 2 or more of: ESAT-6, CFP-10, Rv3873 and Rv3878, are provided to the sample.

9. A method according to any one of the preceding claims wherein recognition by the T cells is measured by detection of production of a cytokine in the T cell and/or secretion of a cytokine from the T cell.

10. A method according to claim 9 wherein detection of the cytokine is carried out by using antibodies to the cytokine, wherein the antibodies are optionally immobilised and/or the cytokine is optionally IFN-γ or IL-2.

11. A method according to any one of the preceding claims wherein the T cells which are tested are freshly isolated (non-cultured) T cells or T cells after being frozen.

12. A method according to any one claims 1 to 10, wherein the T cells which are tested have been cultured *in vitro.*

13. Use in a method according to any one of the preceding claims of a kit comprising means for determining whether the individual has a T cell response to one or more of the following mycobacterial antigens:
- CFP-10,
- Rv3873, or
- Rv3878.

14. Use according to claim 13, wherein the kit further comprises one or more peptides from CFP-10, Rv3873 and/or Rv3878.

15. Use according to claim 13 or claim 14, wherein the kit further comprises antibodies to IFN-γ or IL-2 immobilised on a solid support, and/or peptides from ESAT-6.

## Patentansprüche

1. Verfahren zum Detektieren, ob sich bei einem Individuum eine aktive mykobakterielle Erkrankung entwickeln wird, wobei das Verfahren umfasst:
Feststellen, ob die T-Zellen bei einem Individuum auf ein oder mehre der nachstehenden mykobakteriellen Antigene ansprechen:
- CFP-10,
- Rv3873 oder
- Rv3878.

2. Verfahren nach Anspruch 1, bei dem die Feststellung, ob die T-Zellen bei einem Individuum auf das mykobakterielle Antigen ansprechen, ausgeführt wird, indem bewertet wird:
- ob die T-Zellen bei einem Individuum das mykobakterielle Antigen erkennen, und/oder
- das Niveau der T-Zellen, welche für dieses mykobakterielle Antigen in einer Probe des Individuums spezifisch sind.

3. Verfahren nach Anspruch 1 oder 2, umfassend:
Feststellen, ob die T-Zellen des Individuums auf 2, 3, 4 oder sämtliche der Nachstehenden: ESAT-6, CFP-10, Rv3873 oder Rv3878 ansprechen.

4. Verfahren nach Anspruch 3, umfassend:
Feststellen, ob die T-Zellen des Individuums auf ESAT-6 und CFP-10 ansprechen.

5. Verfahren nach einem der vorgenannten Ansprüche, bei dem T-Zellen des Individuums mit einem Peptid in Kontakt gebracht werden, das ein T-Zellen-Epitop von ESAT-6, CFP-10, Rv3873 oder Rv3878 umfasst; und das Ansprechen der T-Zellen auf das Peptid bewertet wird.

6. Verfahren nach einem der vorgenannten Ansprüche, umfassend:
Feststellung der T-Zellen-Erkennung eines Peptids, umfassend: SEQ ID NO:1, SEQ ID NO:2, SEQ ID NO:4, SEQ ID NO:5 oder SEQ ID NO:6 oder eines Homologs einer dieser Sequenzen, das zumindest eine 70%ige Homologie dazu aufweist.

7. Verfahren nach einem der Ansprüche 1 bis 5, umfassend: Feststellung einer T-Zellen-Erkennung eines Fragments und/oder eines Homologs von ESAT-6, CFP-10, Rv3873 oder Rv3878, wobei das Fragment und/oder Homolog von einer T-Zelle erkannt werden kann, die ein T-Zellen-Epitop von ESAT-6, CFP-10, Rv3873 oder Rv3878 erkennt.

8. Verfahren nach einem der vorstehenden Ansprüche, bei dem die T-Zellen in einer Probe des Individuums vorhanden sind, und
- sämtliche zu prüfenden Peptide gleichzeitig für die Probe bereitgestellt werden,
und/oder
weniger als 20 und bevorzugt weniger als 10 unterschiedliche Peptide für die Probe bereitgestellt werden,
und/oder
mindestens 1 oder 2 Peptide von 2 oder mehreren von ESAT-6, CFP-10, Rv3873 und Rv3878 für die Probe bereitgestellt werden.

9. Verfahren nach einem der vorherigen Ansprüche, bei dem die T-Zellen-Erkennung durch Detektion der Bildung eines Zytokins in der T-Zelle und/oder Sekretion eines Zytokins aus der T-Zelle gemessen wird.

10. Verfahren nach Anspruch 9, bei dem die Detektion des Zytokins durch Verwendung von Zytokin-Antikörpern ausgeführt wird, wobei die Antikörper wahlweise immobilisiert sind und/oder es sich bei dem Zytokin wahlweise um IFN-γ oder IL-2 handelt.

11. Verfahren bei einem der vorherigen Ansprüche, bei dem die zu prüfenden T-Zellen frisch isolierte (nicht-kultivierte) T-Zellen oder eingefrorene T-Zellen sind.

12. Verfahren nach einem der Ansprüche 1 bis 10, bei dem die zu prüfenden T-Zellen *in vitro* kultiviert sind.

13. Verwendung eines Kits bei einem Verfahren nach einem der vorherigen Ansprüche, umfassend: Mittel zum Feststellen, ob die T-Zellen bei einem Individuum auf ein oder mehre der nachstehenden mykobakteriellen Antigene ansprechen:
- CFP-10,
- Rv3873 oder
- Rv3878.

14. Verwendung nach Anspruch 13, bei der das Kit weiterhin ein Peptid oder mehrere Peptide von CFP-10, Rv3873 und/oder Rv3878 umfasst.

15. Verwendung nach Anspruch 13 oder Anspruch 14, bei der das Kit weiterhin umfasst: IFN-γ- oder IL-2-Antikörper, die auf einem festen Träger immobilisiert sind, und/oder Peptide von ESAT-6.

## Revendications

1. Procédé permettant de détecter si un individu va ou non développer une maladie mycobactérienne active comprenant la détermination du fait que ledit individu produit ou non une réponse des cellules T à un ou plusieurs des antigènes mycobactériens suivants :
- CFP-10,
- Rv3873, ou
- Rv3878.

2. Procédé selon la revendication 1, dans lequel la détermination du fait que l'individu produit ou non une réponse des cellules T à l'antigène mycobactérien est réalisée par évaluation :
- du fait que les cellules T de l'individu reconnaissent ou non ledit antigène mycobactérien, et/ou
- du niveau des cellules T qui sont spécifiques dudit antigène mycobactérien dans un échantillon de l'individu.

3. Procédé selon la revendication 1 ou 2, comprenant la détermination du fait que l'individu produit ou non une réponse des cellules T à 2, 3, 4 ou la totalité de : ESAT-6, CFP-10, Rv3873 ou Rv3878.

4. Procédé selon la revendication 3, comprenant la détermination du fait que l'individu produit ou non une réponse des cellules T à ESAT-6 et à CFP-10.

5. Procédé selon l'une quelconque des revendications précédentes, dans lequel les cellules T de l'individu sont mises en contact avec un peptide qui comprend un épitope des cellules T d'ESAT-6, de CFP-10, de Rv3873 ou de Rv3878 ; et la réponse des cellules T au peptide est évaluée.

6. Procédé selon l'une quelconque des revendications précédentes, comprenant la détermination de la reconnaissance par les cellules T d'un peptide comprenant SEQ ID NO : 1, SEQ ID NO : 2, SEQ ID NO : 4, SEQ ID NO : 5, ou SEQ ID NO : 6, ou un homologue de l'une desdites séquences qui présente une homologie d'au moins 70 % avec celles-ci.

7. Procédé selon l'une quelconque des revendications 1 à 5, comprenant la détermination de la reconnaissance par les cellules T d'un fragment et/ou d'un homologue d'ESAT-6, de CFP-10, de Rv3873 ou de Rv3878, dans lequel ledit fragment et/ou ledit homologue peuvent être reconnus par une cellule T qui reconnaît un épitope des cellules T d'ESAT-6, de CFP-10, de Rv3873 ou de Rv3878.

8. Procédé selon l'une quelconque des revendications précédentes, dans lequel les cellules T sont présentes dans un échantillon de l'individu, et
- la totalité des peptides qui doivent être testés sont ajoutés simultanément à l'échantillon, et/ou
- moins de 20, et de préférence moins de 10, peptides différents sont ajoutés à l'échantillon, et/ou
- au moins 1 ou 2 peptides de 2 ou plus de : ESAT-6, CFP-10, Rv3873 et Rv3878, sont ajoutés à l'échantillon.

9. Procédé selon l'une quelconque des revendications précédentes, dans lequel la reconnaissance par les cellules T est mesurée par détection de la production d'une cytokine dans la cellule T et/ou par la sécrétion d'une cytokine par la cellule T.

10. Procédé selon la revendication 9, dans lequel la détection de la cytokine est réalisée en utilisant des anticorps anti-cytokine, dans lequel les anticorps sont facultativement immobilisés et/ou la cytokine est facultativement l'IFN-γ ou l'IL-2.

11. Procédé selon l'une quelconque des revendications précédentes, dans lequel les cellules T qui sont testées sont des cellules T fraîchement isolées (non cultivées) ou des cellules T qui ont été congelées.

12. Procédé selon l'une quelconque des revendications 1 à 10, dans lequel les cellules T qui sont testées ont été cultivées *in vitro.*

13. Utilisation dans un procédé selon l'une quelconque des revendications précédentes d'un kit qui comprend un moyen de détermination du fait que l'individu produit ou non une réponse des cellules T à un ou plusieurs des antigènes mycobactériens suivants :
- CFP-10,
- Rv3873, ou
- Rv3878.

14. Utilisation selon la revendication 13, dans laquelle le kit comprend en outre un ou plusieurs peptides de CFP-10, Rv3873 et/ou Rv3878.

15. Utilisation selon la revendication 13 ou la revendication 14, dans laquelle le kit comprend en outre des anticorps anti-IFN-y ou IL-2 immobilisés sur un support solide, et/ou des peptides d'ESAT-6.
